# EUROPEAN PATENT SPECIFICATION

(11) **EP 1 502 953 B1**
(45) Date of publication and mention of the grant of the patent: **28.01.2009**
(21) Application number: 03017114.4
(22) Date of filing: 28.07.2003
(51) Int. Cl.: C12N 15/82, C12Q 1/68, A01H 5/00

(54) **Chemically-inducible promoters for the expression of proteins in plants**
Chemisch induzierbare Promotoren zum kontrollieren der Expression von Proteinen in Pflanzen
Promoteurs chimiquement inductibles pour controler l'expression des proteines dans des plantes

(43) Date of publication of application: 02.02.2005
(73) Proprietor: Universität Hohenheim, 70593 Stuttgart (DE)
(72) Inventor: Pfitzner, Artur, Prof. Dr., 70567 Stuttgart (DE); Roth, Bernhard, 70184 Stuttgart (DE)
(74) Representative: Müller-Boré & Partner Patentanwälte

(56) References cited:
- WO-A-95/19443
- WO-A-98/03536
- DATABASE EMBL 26 April 2000 (2000-04-26), FEDERSPIEL N.A. ET AL: "Arabidopsis thaliana chromosome I BAC T6A9 genomic sequence, complete sequence." XP002264770 Database accession no. AC064879
- DATABASE EMBL 7 February 2000 (2000-02-07), FEDERSPIEL N.A. ET AL: "Arabidopsis thaliana chromosome I BAC T14P4 genomic sequence, complete sequence." XP002264771 Database accession no. AC022521
- DATABASE EMBL 7 September 2002 (2002-09-07), ALONSO J.M. ET AL: "SALK_053252.47.90.x Arabidopsis thaliana TDNA insertion lines Arabidopsis thaliana genomic clone SALK_053252.47.90.x, DNA sequence." XP002264772 Database accession no. BH909376
- WEIGEL R R ET AL: "NIMIN-1, NIMIN-2 and NIMIN-3, members of a novel family of proteins from Arabidopsis that interact with NPR1/NIM1, a key regulator of systemic acquired resistance in plants." PLANT MOLECULAR BIOLOGY. NETHERLANDS MAY 2001, vol. 46, no. 2, May 2001 (2001-05), pages 143-160, XP002265148 ISSN: 0167-4412
- DATABASE EMBL 18 July 1997 (1997-07-18), NAKAMURA Y.: "Arabidopsis thaliana genomic DNA, chromosome 5, P1 clone:MLN1." XP002265149 Database accession no. AB005239
- DATABASE EMBL 30 November 1999 (1999-11-30), CHOISNE N. ET AL: "Arabidopsis thaliana DNA chromosome 3, BAC clone F18L15" XP002264773 Database accession no. AL133298
- DATABASE EMBL 11 June 1999 (1999-06-11), SATO S. ET AL: "Arabidopsis thaliana genomic DNA, chromosome 3, TAC clone:K20I9." XP002265150 Database accession no. AB028608

## Description

The present invention relates to a nucleic acid containing selectively inducible regulatory sequences, in particular promoters, for a controlled expression of desired expression products in suitable host expression organisms such as transgenic plants. Further, the present invention relates to a method for detecting the activity of a regulatory sequence in suitable cells, wherein a transgene comprises a potential regulatory sequence operably linked to the Bax gene or a functional derivative thereof and the Bax expression correlates with the activity of the regulatory sequence in said cells.

With reference to industrial and medical applications (e.g. enzymes for technical uses, therapeutical or diagnostic antibodies, vaccines) the expression of proteins in transgenic plants is becoming more and more interesting for pharmaceutical and chemical companies. The major reasons are security and costs. Traditionally, valuable proteins for pharmaceutical applications (hormones, antibodies etc.) are produced in cultivated animal cells. Since most cultivation media contain 10% or more fetal calf serum (FCS), there is always the danger of contamination with BSE causing agents. In addition, animal tissue cultures can be contaminated with viruses which may be transmitted to humans. Furthermore, because of the FCS and other additives, the cultivation media are often very expensive. Additional costs come from the high energy requirement for temperature control of large cultivation entities. This is also true for the production of recombinant proteins in microorganisms.

Cultivation of plants is cheap because only sun light, water and some inorganic nutritions are necessary to support plant growth. In addition, plants can be grown on fields in almost unlimited quantity. Therefore, there are no scale-up problems. However, one essential requirement for the successful production of a heterologous protein in a transgenic organism is the combination of the respective transgene with strong and reliable regulatory elements, the promoters. Promoters lead to an efficient synthesis of the transgenic mRNA, which is then translated to the protein.

Further, WO 95/19443 describes the isolation, cloning and identification of novel cDNA clones coding for pathogenesis-related proteins.

For the production of recombinant proteins in transgenic plants mostly the 35S RNA promoter is used (Pietrzak et al. (1987) Nucleic Acids Res. 14:5857). The 35S promoter was originally isolated from the cauliflower mosaic virus (CaMV) and leads to a high and constitutive synthesis of a transcript for the desired genes. Other promoters, which are less frequent in use are the 19S RNA promoter from CaMV (Paszkowski et al. (1985) Mol. Gen. Genet. 199:178) and the promoter from the nopaline synthase gene from *Agrobacterium tumefaciens* (An et al. (1984) EMBO J. 4:277).

All these promoters are active during the whole life of a plant and therefore the recombinant protein is synthesized all the time. If these proteins continuously accumulate during the development of the plant, they can be degraded or modified and accordingly loose their biological activity. In most cases, the expressed protein is harmful or even lethal to the particular plant. This can be a major obstacle in establishing transgenic plants with high expression levels. In this case, it is not possible to obtain any transgenic plant at all, or only plants which show very low levels of protein expression.

Therefore, it would be highly desirable to posses a promoter which can be switched on specifically at any time of the plant development by treatment of the plant with chemicals. Transgenic plants could be grown to an appropriate size and then the expression of the recombinant protein could be induced to a high level for a short time. In that way problems like protein degradation or killing of the plants by the heterologous protein could be avoided.

The most prominent plant promoters which can be induced by chemicals are the promoters of genes encoding pathogenesis-related (PR) proteins. PR genes are induced during the plant's defence reaction against pathogens. They are switched off most of the time during the development of the plant and they can be induced by the plant hormone salicylic acid. The major problem with these promoters comes from the fact that they are also induced by a developmental stimulus shortly before flowering of the plants (Grüner and Pfitzner (1994) Eur. J. Biochem. 220:247). Therefore, it is hardly possible to get seed material from plants expressing proteins which are harmful to the particular organism.

Furthermore, for the protein production in transgenic plants it is necessary to know in which parts of the plants the regulatory regions, in particular promoters, of the transgenes are active and inducible, respectively. In order to analyse the regulatory regions in living plants, it is necessary to express reporter genes under the control of these regulatory regions. At present, the reporter genes GUS (β-Glucoronidase), Luc (luciferase) and GFP (green fluorescent protein) are used in plants. However, these reporter genes are not very sensitive and the analysis of whole plants over the complete lifetime is not possible. Further, in contrast to animal cells, plant cells possess a rigid cell wall. Therefore, the substrate application for the reporter genes GUS and Luc is difficult, defective, takes a lot of time and is expensive. Moreover, the use of GFP in plants is difficult due to their autofluorescence. At present, no reporter gene is available to detect sensitive, easily and over the whole lifetime of a plant the activity of regulatory regions.

Thus, the technical problem underlying the present invention is to provide regulatory sequences, particularly for plants, which can be selectively switched on by externally added chemicals thereby inducing the expression of transgenes, wherein the regulatory sequences should not be switched on during the normal development of e.g. the plant. A further object of the present invention is to provide a method for detecting the activity of a regulatory sequence in plants or parts thereof, wherein the regulatory activity is easily detectable over the whole lifetime of the plant.

The solution to the above technical problems is achieved by the embodiments characterized in the claims.

In particular, the present invention relates to a nucleic acid containing the nucleotide sequence SEQ-ID-No. 1 which is operably linked to a transgene.

The nucleotide sequence SEQ-ID-No. 1 comprises a regulatory sequence, in particular a promoter sequence. The term "regulatory sequence" includes a nucleotide sequence containing elements necessary for the expression/transcription of a desired product. The regulatory sequence may be a regulatory element which does not constitutively express e.g. a gene but is selectively inducible by chemicals resulting in an expression of e.g. a gene upon induction. The wording "no constitutive expression" means that the regulatory sequence such as a promoter sequence is preferably completely inactive prior to any measure resulting in an induction thereof. In a preferred embodiment of the present invention, the first nucleotide sequence is an inducible plant promoter.

In a preferred embodiment of the present invention the chemicals are selected from the group consisting of organic compounds. More preferably the chemicals are selected from the group consisting of phenolic compounds, thiamine, benzoic acid, isonicotinic acid (INA), and derivatives thereof. The phenolic compounds are for example salicylic acid or structural or functional related derivatives thereof.

The above defined nucleic acid SEQ-ID No.1 is operably linked to a second nucleotide sequence comprising a desired sequence (herein also designated as "transgene") to be expressed such as a functional gene. There is no particular limitation of the sequence to be expressed, and upon expression in a suitable host organism the sequence results in e.g. a polypeptide, a protein or a RNA molecule.

The term "operably linked" means that the first and second nucleotide sequence are connected to each other in such a way that the first nucleotide sequence controls the transcription of the second nucleotide sequence.

The present invention also relates to vectors comprising the above-defined nucleic acid according to the invention. Examples of vectors are pBin19, pGC4-00, pUC18/19, pBluescript.

The present invention also relates to a host organism comprising the nucleic acid according to the invention or the vector according to the invention. The host organism is for example a prokaryotic organism such as *E. coli* or *Agrobacterium tumefaciens,* or an eukaryotic organism such as a plant cell (including a transgenic plant). The nucleic acid according to the present invention can be also contained in a virus or viroid suitable for the preparation of a transgenic organism such as a transgenic plant.

Further, the present invention relates to the use of the above-defined nucleic acid for the preparation of a transgenic plant whose transgene is selectively inducible by exogenously added chemicals. The term "exogenously added chemicals" includes chemicals that are not produced by the plant itself (or produced only in amounts which do not substantially cause induction of the above-defined regulatory sequence), and chemicals that are produced by the plant at some time during the life-time. The term "selectively inducible" means that the expression of the transgene is substantially induced ("switched on") only by adding specific chemicals.

The present invention further relates to a transgenic plant such as a tobacco plant, tomato plant or potato plant comprising the nucleic acid sequence according to the present invention for the purpose of a regulated expression of any homologous or heterologous transgene. The term "transgenic plant" includes the whole plant as such and parts thereof, such as root, stem, leaf, organ-specific tissue or cells, the reproductive material thereof, especially seeds, and the seedlings thereof. The term "heterologous transgene" means a gene which stems from a source other than the wildtype of the transgenic plant. The term "homologous transgene" means a gene which stems from the wildtype of the transgenic plant. In one embodiment of the present invention the transgenic plant contains the above-defined nucleic acid of the invention stably integrated into the genetic material. Alternatively, the transgenic plant expresses transiently the desired sequence contained in the above-defined nucleic acid.

In a preferred embodiment of the present invention the expression of the above-defined transgene is induced after treatment with chemicals of the transgenic plant.

Surprisingly, the nucleic acid according to the present invention is capable to induce selectively the expression of a desired sequence ("transgene") in e.g. transgenic plants after treatment with certain chemicals. Accordingly, it is possible to grow the transgenic plants to a size sufficient to obtain enough plant material, and then to induce the expression of a transgene resulting in the desired product such as e.g. a polypeptide, a protein or a RNA molecule.

The nucleic acid according to the present invention and the vector according to the present invention can be prepared by means of prior art methods. This includes also methods for cultivating suitable host cells and recovering said nucleic acids or said vectors from said hosts cells and/or the culture medium.

The figures show:
Figure 1 shows the nucleotide sequence of the NIMIN-1 promoter (SEQ-ID-No.1).
Figure 2 shows the nucleotide sequence of the NIMIN-2 promoter (SEQ-ID-No.2).
Figure 3 shows a bar diagram comparing the GUS (beta-glucoronidase) activity of PR1a-GUS (321-9) and NIMIN2-GUS (322-7) fusion constructs in transgenic plants after induction with salicylic acid (SA) or Bion, wherein the GUS-activity of water-treated fusion-constructs in transgenic plants is used as background control.
Figure 4 shows a bar diagram which correlates the amount of SA used for the treatment of the plant with the GUS activity in two independent NIMIN2-GUS transgenic plants (322-2 and 322-7) after induction with salicylic acid (SA), wherein the GUS-activity of water-treated fusion-constructs in transgenic plants is used as background control.
Figure 5 shows a Western Blot which correlates the amount of SA used for the treatment of the plant with the expression of the endogenous PR-1 proteins in transgenic plant 322-2 as depicted in Fig. 4, and which is used as a positive control.
Figure 6 shows the spontaneous expression of the Bax gene under the control of the PR-1a promoter (A) in leaves and (B) on the stem of transgenic tobacco plants.
Figure 7 shows plants which contain the human Bax gene under the control of the PR-1a promoter (A), the NIMIN-2 promoter (B) or the NIMIN-1 promoter (C and D).

The present invention will now be further illustrated in the following examples, without being limited thereto.

### EXAMPLES

### Example 1: Isolation of the NIMIN-1 and NIMIN-2 promoters

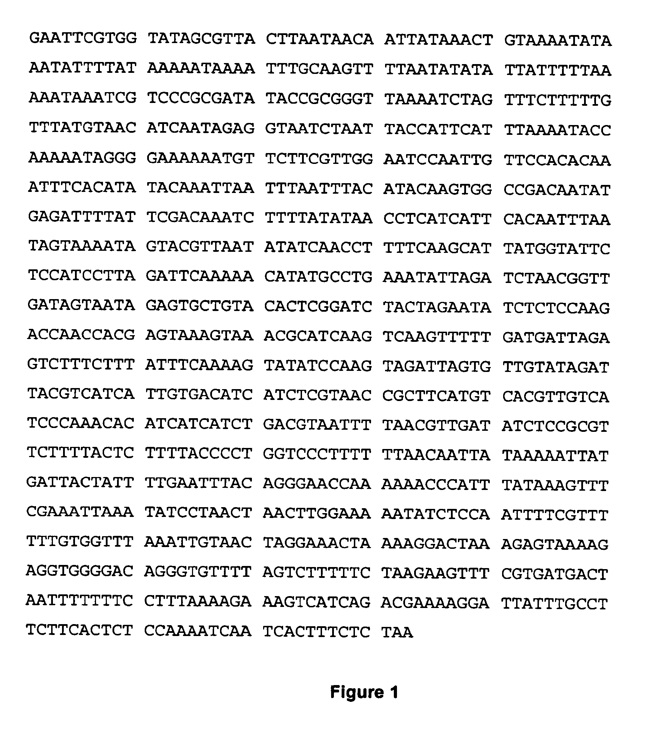

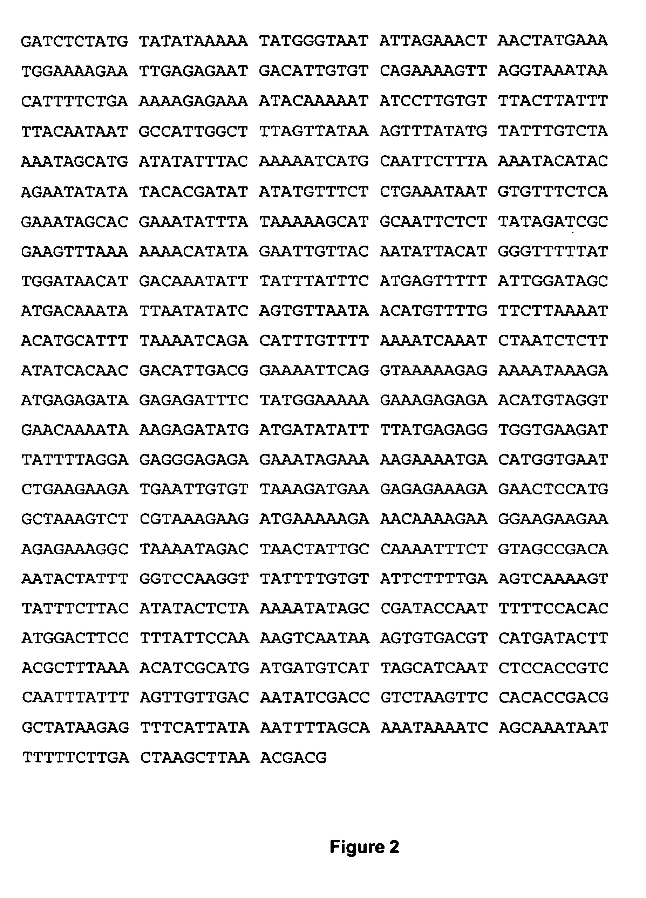

The cDNAs for the NIMIN proteins NIMIN-1, NIMIN-2 and NIMIN-3 have been isolated as new interacting proteins with the regulatory NPR1 protein in *Arabidopsis thaliana* (Weigel et al. (2001) Plant Mol. Biol. 46:143). The mRNAs for the NIMIN-1 and NIMIN-2 proteins were shown to be inducible by salicylic acid and Bion, a substance which can replace salicylic acid in some of its functions (Weigel et al. (2001) Plant Mol. Biol. 46:143). To identify the regulatory elements responsible for this induction, the promoter elements were amplified by PCR from genomic DNA of *Arabidopsis thaliana* using the primers N1-P1 (SEQ-ID-No. 3) and N1-P2 (SEQ-ID-No. 4) for NIMIN-1 and N2-P1 (SEQ-ID-No.5) and N2-P2 (SEQ-ID-No.6) for NIMIN-2 (N1-P1: 5'-CCAAGCTTGTCTCATGAATTCGTGGTATAGCG-3'; N1-P2: 5'-CCGGATCCTTAGAGAAAGTGATTGATTTTGG-3'; N2-P1: 5'-CCCCACGTTAACGATGATCAC-3'; N2-P2: 5'-CTGGATCCCGTCGTTTAAGCTTAGTCAA-3'); The respective PCR fragments were analysed on an agarose gel and yielded the expected sizes (1.1 kb for NIMIN-1 and 1.2 kb for NIMIN-2). The fragments were cut with the restriction enzymes Hindlll and BamHI (NIMIN-1 promoter element) or BgIII and BamHI (NIMIN-2 promoter element), and ligated in the cloning vector pUC19. The resulting clones were sequenced and the nucleotide sequence is shown in Figure 1 (NIMIN-1) and Figure 2 (NIMIN-2).

### Example 2: Construction of reporter gene fusions and generation of transgenic plants:

The DNA fragments containing the respective promoter sequences were excised from the pUC19 subclones and ligated in pUC/0-GUS (Beilmann et al. (1991) Eur. J. Biochem. 196:415). The resulting ligation products were analysed by a restriction enzyme digest with EcoRI (NIMIN1-GUS) or Xbal and EcoRI (NIMIN2-GUS). The 3.2 kb band, representing the reporter gene-promoter fusion was excised from an agarose gel and ligated in the *Agrobacterium* vector pBin19, yielding pBin19/N1-GUS and pBin19/N2-GUS. The plasmids were moved from *E. coli* into *Agrobacterium tumefaciens* via triparental mating.

The resulting *Agrobacteria* strains were used to generate transgenic plants containing the reporter gene constructs.

### Example 3: Characterisation of the promoter activities of the NIMIN promoters

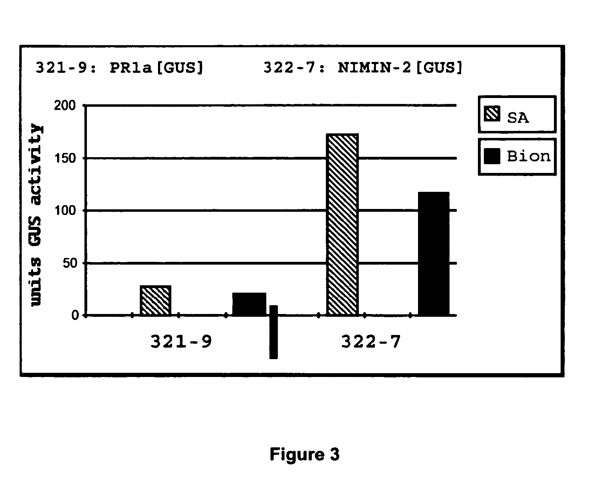

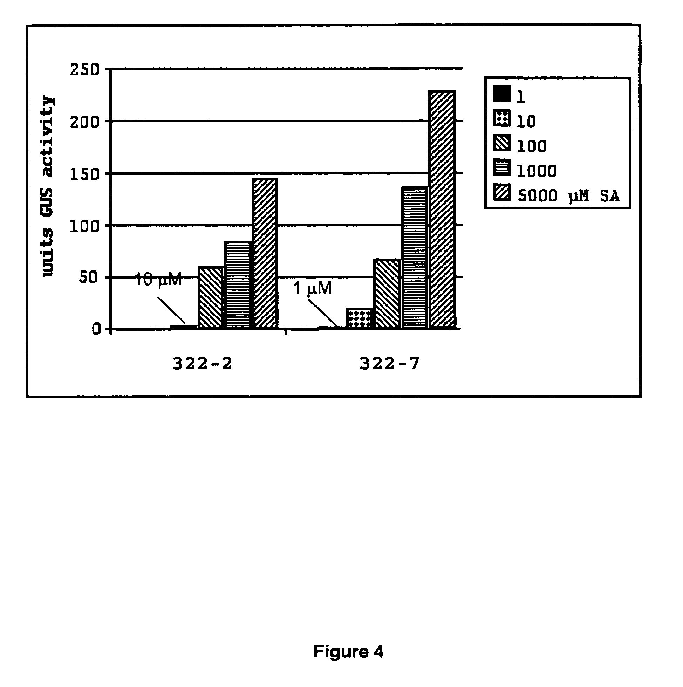

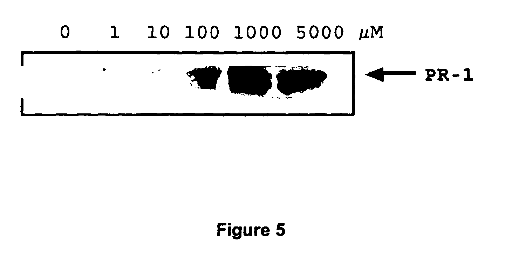

To characterise the influence of chemicals on the activity of the NIMIN promoters, leaf discs were punched from the transgenic lines NIMIN1-GUS and NIMIN2-GUS. As a control, PR-1 a promoter fusions were used. The leaf discs were floated on 1 mM SA, 140 mg/l Bion (a functional analogue to salicylic acid) or water. After 3 days, protein extracts were prepared and the activity of the GUS reporter gene in the extracts was assayed. The resulting reporter gene activities were 5 times higher in the NIMIN-2 promoter fusions than in PR-1a promoter fusions (cf. Figure 3). Further, leaf discs from the transgenic lines NIMIN1-GUS and NIMIN2-GUS were incubated with different salicylic acid concentrations. The activity of the NIMIN promoters depends on the salicyclic acid concentration (cf. Figures 4 and 5).

### Example 4: Construction of a reporter gene (Bax) fusion with the PR-1a promoter and generation of transgenic tobacco plants - Reference Example

The vector pUC19/His(Bax) was digested with the restriction enzymes Sacl and Bgl II. The resulting DNA fragment containing the respective human Bax alpha gene with an N-terminal 6x His tag was ligated in the binary vector pBin19/PR1a-GUS (Beilmann et al. (1992) Plant Mol. Biol. 18:65), in which the GUS was excised before with restriction enzymes Sacl and BamHl. The ligation product was transformed in *E. coli* DH5α, and the resulting colonies were analyzed by a restriction enzyme digest with EcoRI and Hindlll and PCR, yielding the vector pBin19/PR1a-Bax. The plasmid was moved from *E. coli* to *Agrobacterium tumefaciens* LBA4404 via electroporation. The resulting *Agrobacterium* strain was used to generate transgenic tobacco plants *(Nicotiana tabacum* var. Samsun NN/nn) containing the reporter gene construct.

### Example 5: Analysis of the regulation of the NIMIN promoters by endogenous stimuli

The most important feature of an inducible promoter is not only the inducibility by exogenous substances but also that the promoter is switched off under normal conditions. To investigate, if the NIMIN promoters fulfil these requirements, transgenic tobacco plants with NIMIN promoter-Bax fusions were generated as described above. Bax is lethal for any cell. Therefore, any leakiness of the NIMIN promoter should lead to necrotic areas in the plants. Plants were grown in the green house and monitored for their phenotype.

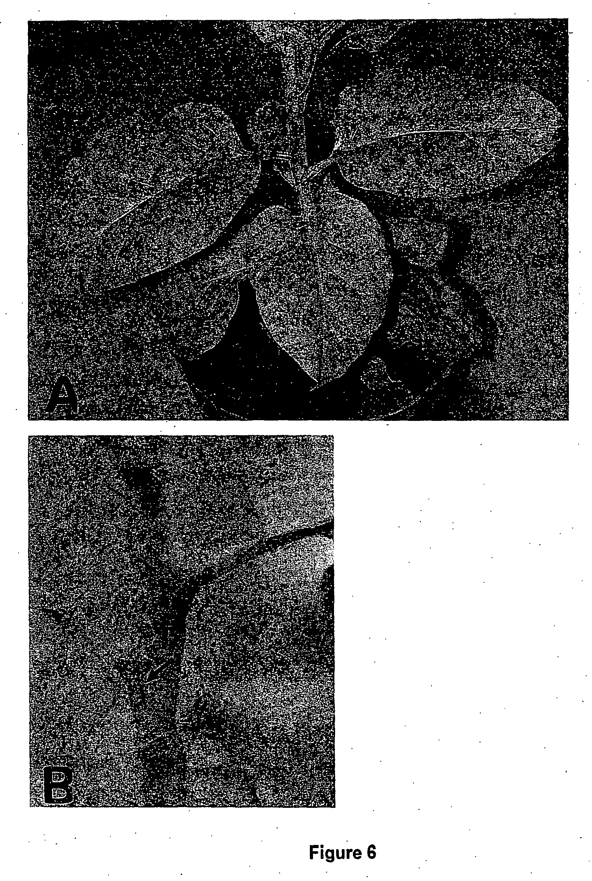

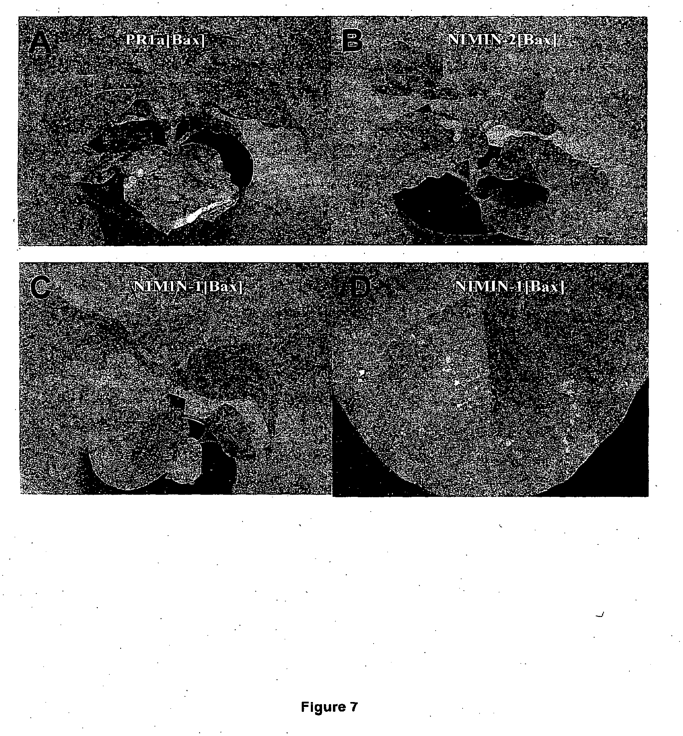

While use of the PR-1a promoter resulted in major necrotic regions later in the development of the plants (cf. Figure 6 and Figure 7A), there were no necrotic areas on the NIMIN2-Bax plants and only minor lesions on the lower leaves of NIMIN1-Bax plants (cf. Figure 7B-D).

## Claims

1. A nucleic acid containing the nucleotide sequence SEQ-ID-No. 1 which is operably linked to a transgene.

2. The nucleic acid according to claim 1, wherein the nucleotide sequence is a regulatory sequence.

3. The nucleic acid according to claim 2, wherein the regulatory sequence is a promoter sequence selectively inducible by chemicals.

4. The nucleic acid according to claim 3, wherein the chemicals are selected from the group consisting of organic compounds.

5. The nucleic acid according to claim 4, wherein the organic compounds are selected from the group consisting of phenolic compounds, thiamine, benzoic acid, isonicotinic acid (INA), and derivatives thereof.

6. The nucleic acid according to claim 5, wherein the phenolic compound is salicylic acid or a structural or functional derivative thereof.

7. A vector containing the nucleic acid according to anyone of claims 1 to 6.

8. A non-human host organism containing the nucleic acid according to anyone of claims 1 to 6 or the vector according to claim 7.

9. The host organism according to claim 8, which is selected from the group consisting of a bacteria cell or a plant cell.

10. A transgenic plant containing at least the nucleic acid according to anyone of claims 1 to 6.

11. The transgenic plant according to claim 10, wherein the nucleic acid is stably integrated into the genetic material.

12. The transgenic plant according to claim 10 or 11, wherein the transgene is transiently expressed.

13. The transgenic plant according to anyone of claims 10 to 12, wherein the expression of the transgene is selectively induced upon treatment with chemicals.

14. The transgenic plant according to claim 13, wherein the chemicals are selected from the group consisting of organic compounds as defined in anyone of claims 4 to 6.

## Patentansprüche

1. Nukleinsäure, enthaltend die Nukleotidsequenz SEQ ID NR. 1, die funktionell mit einem Transgen verbunden ist.

2. Nukleinsäure nach Anspruch 1, wobei die Nukleotidsequenz eine regulatorische Sequenz ist.

3. Nukleinsäure nach Anspruch 2, wobei die regulatorische Sequenz eine selektiv durch Chemikalien induzierbare Promotorsequenz ist.

4. Nukleinsäure nach Anspruch 3, wobei die Chemikalien aus der Gruppe, bestehend aus organischen Verbindungen ausgewählt sind.

5. Nukleinsäure nach Anspruch 4, wobei die organischen Verbindungen aus der Gruppe, bestehend aus Phenolverbindungen, Thiamin, Benzoesäure, Isonicotinsäure (INA) und Derivaten davon ausgewählt sind.

6. Nukleinsäure nach Anspruch 5, wobei die Phenolverbindung Salicylsäure oder ein strukturelles oder funktionelles Derivat davon ist.

7. Vektor, enthaltend die Nukleinsäure nach einem der Ansprüche 1 bis 6.

8. Nicht-humaner Wirtsorganismus, enthaltend die Nukleinsäure nach einem der Ansprüche 1 bis 6 oder den Vektor nach Anspruch 7.

9. Wirtsorganismus nach Anspruch 8, der aus der Gruppe, bestehend aus einer Bakterienzelle oder einer Pflanzenzelle ausgewählt ist.

10. Transgene Pflanze, enthaltend mindestens die Nukleinsäure nach einem der Ansprüche 1 bis 6.

11. Transgene Pflanze nach Anspruch 10, wobei die Nukleinsäure stabil in das genetische Material integriert ist.

12. Transgene Pflanze nach Anspruch 10 oder 11, wobei das Transgen transient exprimiert wird.

13. Transgene Pflanze nach einem der Ansprüche 10 bis 12, wobei die Expression des Transgens bei Behandlung mit Chemikalien selektiv induziert wird.

14. Transgene Pflanze nach Anspruch 13, wobei die Chemikalien aus der Gruppe, bestehend aus wie in einem der Ansprüche 4 bis 6 definierten organischen Verbindungen ausgewählt sind.

## Revendications

1. Acide nucléique contenant la séquence nucléotidique SEQ-ID-N° 1 qui est opérationnellement liée à un transgène.

2. Acide nucléique selon la revendication 1, dans lequel la séquence nucléotidique est une séquence de régulation.

3. Acide nucléique selon la revendication 2, dans lequel la séquence de régulation est une séquence promoteur sélectivement inductible par des substances chimiques.

4. Acide nucléique selon la revendication 3, dans lequel les substances chimiques sont choisies dans l'ensemble constitué de composés organiques.

5. Acide nucléique selon la revendication 4, dans lequel les composés organiques sont choisis dans l'ensemble constitué de composés phénoliques, de thiamine, d'acide benzoïque, d'acide isonicotinique (INA) et de dérivés de ceux-ci.

6. Acide nucléique selon la revendication 5, dans lequel le composé phénolique est l'acide salicylique ou un dérivé structurel ou fonctionnel de celui-ci.

7. Vecteur contenant l'acide nucléique selon l'une quelconque des revendications 1 à 6.

8. Organisme hôte non humain contenant l'acide nucléique selon l'une quelconque des revendications 1 à 6 ou le vecteur selon la revendication 7.

9. Organisme hôte selon la revendication 8, qui est choisi dans l'ensemble constitué d'une cellule bactérienne ou d'une cellule végétale.

10. Plante transgénique contenant au moins l'acide nucléique selon l'une quelconque des revendications 1 à 6.

11. Plante transgénique selon la revendication 10, dans laquelle l'acide nucléique est intégré de manière stable dans le matériau génétique.

12. Plante transgénique selon la revendication 10 ou 11, dans laquelle le transgène est exprimé de manière transitoire.

13. Plante transgénique selon l'une quelconque des revendications 10 à 12, dans laquelle l'expression du transgène est induite sélectivement par traitement avec des substances chimiques.

14. Plante transgénique selon la revendication 13, dans laquelle les substances chimiques sont choisies dans l'ensemble constitué de composés organiques tels que définis dans l'une quelconque des revendications 4 à 6.


### SEQUENCE LISTING

<110> Universität Hohenheim
<120> Inducible promoters for the expression of proteins in plants and method for detecting them
<130> H 2269
<160> 6
<170> PatentIn version 3.1
<210> 1
   <211> 1033
   <212> DNA
   <213> Arabidopsis thaliana
<400> 1
   gaattcgtgg tatagcgtta cttaataaca attataaact gtaaaatata aatattttat 60
   aaaaataaaa tttgcaagtt ttaatatata ttatttttaa aaataaatcg tcccgcgata 120
   taccgcgggt taaaatctag tttctttttg tttatgtaac atcaatagag gtaatctaat 180
   taccattcat ttaaaatacc aaaaataggg gaaaaaatgt tcttcgttgg aatccaattg 240
   ttccacacaa atttcacata tacaaattaa tttaatttac atacaagtgg ccgacaatat 300
   gagattttat tcgacaaatc ttttatataa cctcatcatt cacaatttaa tagtaaaata 360
   gtacgttaat atatcaacct tttcaagcat tatggtattc tccatcctta gattcaaaaa 420
   catatgcctg aaatattaga tctaacggtt gatagtaata gagtgctgta cactcggatc 480
   tactagaata tctctccaag accaaccacg agtaaagtaa acgcatcaag tcaagttttt 540
   gatgattaga gtctttcttt atttcaaaag tatatccaag tagattagtg ttgtatagat 600
   tacgtcatca ttgtgacatc atctcgtaac cgcttcatgt cacgttgtca tcccaaacac 660
   atcatcatct gacgtaattt taacgttgat atctccgcgt tcttttactc ttttacccct 720
   ggtccctttt ttaacaatta taaaaattat gattactatt ttgaatttac agggaaccaa 780
   aaaacccatt tataaagttt cgaaattaaa tatcctaact aacttggaaa aatatctcca 840
   attttcgttt tttgtggttt aaattgtaac taggaaacta aaaggactaa agagtaaaag 900
   aggtggggac agggtgtttt agtctttttc taagaagttt cgtgatgact aatttttttc 960
   ctttaaaaga aagtcatcag acgaaaagga ttatttgcct tcttcactct ccaaaatcaa 1020
   tcactttctc taa 1033
<210> 2
   <211> 1226
   <212> DNA
   <213> Arabidopsis thaliana
<400> 2
   gatctctatg tatataaaaa tatgggtaat attagaaact aactatgaaa tggaaaagaa 60
   ttgagagaat gacattgtgt cagaaaagtt aggtaaataa cattttctga aaaagagaaa 120
   atacaaaaat atccttgtgt ttacttattt ttacaataat gccattggct ttagttataa 180
   agtttatatg tatttgtcta aaatagcatg atatatttac aaaaatcatg caattcttta    240
   aaatacatac agaatatata tacacgatat atatgtttct ctgaaataat gtgtttctca    300
   gaaatagcac gaaatattta taaaaagcat gcaattctct tatagatcgc gaagtttaaa    360
   aaaacatata gaattgttac aatattacat gggtttttat tggataacat gacaaatatt    420
   tatttatttc atgagttttt attggatagc atgacaaata ttaatatatc agtgttaata    480
   acatgttttg ttcttaaaat acatgcattt taaaatcaga catttgtttt aaaatcaaat    540
   ctaatctctt atatcacaac gacattgacg gaaaattcag gtaaaaagag aaaataaaga    600
   atgagagata gagagatttc tatggaaaaa gaaagagaga acatgtaggt gaacaaaata    660
   aagagatatg atgatatatt ttatgagagg tggtgaagat tattttagga gagggagaga    720
   gaaatagaaa aagaaaatga catggtgaat ctgaagaaga tgaattgtgt taaagatgaa    780
   gagagaaaga gaactccatg gctaaagtct cgtaaagaag atgaaaaaga aacaaaagaa    840
   ggaagaagaa agagaaaggc taaaatagac taactattgc caaaatttct gtagccgaca    900
   aatactattt ggtccaaggt tattttgtgt attcttttga agtcaaaagt tatttcttac    960
   atatactcta aaaatatagc cgataccaat ttttccacac atggacttcc tttattccaa    1020
   aagtcaataa agtgtgacgt catgatactt acgctttaaa acatcgcatg atgatgtcat   1080
   tagcatcaat ctccaccgtc caatttattt agttgttgac aatatcgacc gtctaagttc   1140
   cacaccgacg gctataagag tttcattata aattttagca aaataaaatc agcaaataat   1200
   tttttcttga ctaagcttaa acgacg                                         1226
<210> 3
   <211> 32
   <212> DNA
   <213> Artificial sequence
<220>
   <223> Primer N1-P1 for the amplification of the NIMIN-1 gene
<400> 3
   ccaagcttgt ctcatgaatt cgtggtatag cg 32
<210> 4
   <211> 31
   <212> DNA
   <213> Artificial sequence
<220>
   <223> Primer N1-P2 for the amplification of the NIMIN-1 gene
<400> 4
   ccggatcctt agagaaagtg attgattttg g 31
<210> 5
   <211> 21
   <212> DNA
   <213> Artificial sequence
<220>
   <223> Primer N2-P1 for the amplification of the NIMIN-2 gene
<400> 5
   ccccacgtta acgatgatca c 21
<210> 6
   <211> 28
   <212> DNA
   <213> Artificial sequence
<220>
   <223> Primer N2-P2 for the amplification of the NIMIN-2 gene
<400> 6
   ctggatcccg tcgtttaagc ttagtcaa 28